(19) Europäisches Patentamt

European Patent Office

Office européen des brevets

(11) Publication number: **0 145 478**
A2

(12) **EUROPEAN PATENT APPLICATION**

(21) Application number: **84308590.3**

(22) Date of filing: **11.12.84**

(51) Int. Cl.⁴: **B 01 J 35/02, B 01 J 23/74, C 07 C 1/04**

(30) Priority: **13.12.83 GB 8333230**

(43) Date of publication of application: **19.06.85 Bulletin 85/25**

(84) Designated Contracting States: **DE FR NL**

(71) Applicant: **Coal Industry (Patents) Limited, Hobart House Grosvenor Place, London SW1X 7AE (GB)**

(72) Inventor: **Thomas, John Merino, 37 Sedley Taylor Road, Cambridge Cambridgeshire CB2 2PN (GB)**
Inventor: **Jones, William, 56 Lantree Crescent, Cambridge Cambridgeshire CB2 2NJ (GB)**
Inventor: **Schlogl, Robert, 5 Dayedenstrasse, CH-4056 Basel (CH)**

(74) Representative: **Wood, John Irwin, NATIONAL COAL BOARD Hobart House Grosvenor Place, London SW1X 7AE (GB)**

(54) **Improvements in catalysts.**

(57) A catalyst having stability in air, exhibiting long life and capable of use in the conversion of synthesis gas to hydrocarbons, consists of particles of a disordered residue intercalate having a catalytic substance derived from the guest substance on the outer surfaces of the particles. The disordered residue intercalate retains about 1–10% of guest substance, modifying its electronic properties.

EP 0 145 478 A2

1

## IMPROVEMENTS IN CATALYSTS

This invention concerns improvements in catalysts, and more particularly concerns catalysts derived from graphite intercalates.

Graphite intercalation compounds, in which guest atoms or molecules are introduced between some or all of the graphite molecular planes, are known, and have been proposed as catalysts. British Patent specification. No. 1 367 112 proposes the use of such complex compounds containing an alkali metal and a transition metal compound as a catalyst for the synthesis of ammonia. It has also been suggested that graphite intercalates could be used in the conversion of synthesis gas into hydrocarbons, but it would appear that such graphite intercalates are unstable in the gaseous environment of the Fischer-Tropsch synthesis, and also in air. It has also been suggested that catalytic activity in graphite intercalates is dependent upon an ordered intercalate structure.

A new class of catalysts has now been designed which makes use of the idea that deliberate prior modification of such intercalates would provide a new way of preparing multiphasic catalysts; especially those containing a modified carbon support, show unexpected stability and unexpected catalytic activity.

The present invention provides a polyphasic multicomponent heterogeneous catalyst comprising particles of a disordered residue intercalate having on the outer surfaces of said particles a finely divided catalytic substance at least partially derived from the precursor guest substance.

The invention also provides a method for the production of the catalysts of the invention, comprising the deintercalation of at least 90% by weight of the guest substance of an intercalate to yield a disordered residue intercalate carrying a finely divided catalytic substance at least partially derived from the guest substance, on the outer surface of said particles and retaining from 1 to 10% of the guest substance in the intercalate matrix.

The intercalates used in the present invention may use graphite, BN, Ta S2 and other layered solids for the matrix; graphite, however, is the preferred material and will be used in the following description without limiting the scope of the invention.

Graphite intercalates used as starting materials may be first, second or higher stage intercalates, and the guest species may be such as to give "acceptor" or "donor" intercalates. The graphite is preferably of relatively small particle size and may be any graphitic form of carbon which can be intercalated; preferred graphites include natural graphite, graphitised carbon black and other types of synthetic graphite, but other materials having at least surface layers of graphitic form carbon may be used. The invention may use intercalates which are saturated (ie. all possible sites for guest species are taken up) or under-saturated.

Both donor intercalates, in which the guest species is an alkali metal, especially potassium, but also rubidium, caesium, sodium or lithium, in which metal donates electrons to the carbon, and acceptor intercalates, in which the guest species is a compound of a metal in Group 4B, 5B, 6B, 7B or Group 8 (the compound accepting electrons from the carbon) may be used in the present invention. In acceptor intercalates, the compound is preferably an oxide, sulfide or especially a halide, particularly a chloride, although carbonyls and other compounds which can form intercalates may be used. The metal is preferably tin, antimony, nickel, or, especially, iron.

In both acceptor and donor intercalates other elements and/or compounds may be co-intercalated, for example in the case of nickel chloride - iron chloride co-intercalates, and such additional metals or compounds may be found to promote the activity of the novel catalyst of the invention and may be used in any suitable amount. The above groups of elements are according to the IUPAC 1965 revision of the Periodic Table.

Intercalates may be prepared in manner known per se; for example, (i) by the contact of the graphite particles with the guest species in the vapour phase, usually at a temperature in the range 250 to 450°C. This may be done using the guest species alone

4

or in the presence of a catalyst or an additive to support intercalation, such as chlorine gas, water vapour, aluminium chloride, iodine or the like; (ii) the intercalate may also be formed using a solvent which upon irradiation allows the formation of radicals from the guest species or from the solvent itself, and this reaction is usually carried out at relatively low temperatures, eg. at 0°C to room temperature, using a saturated solution of the guest species; (iii) or by any other means whereby guest species are inserted into the host structure.

Thus the composition and the method of preparation of the starting material intercalate is not critical; however, it has been found that it is desirable to use the intercalate as soon as possible after preparation in the method of the invention. In particular, it is important that exposure to air of the intercalate be controlled since this, and storage for any prolonged time, even under an inert atmosphere, has been found to affect seriously the catalytic activity of the final catalyst.

The method of the invention achieves partial de-intercalation of the starting material, at least 90% but preferably not more than 99% of the guest substance being de-intercalated and deposited as finely divided particles on the exfoliated carbon support, whether in exactly the same chemical or physical form as the guest substance, or not, on the surface of the graphite particles and in the surface microporous carbon matrix. Thus up to 10%, preferably about 5%, by weight of the original (or modified) guest substance is retained in a disordered residue intercalate, and it is an essential feature of the present invention that this residual intercalation is retained. The residual intercalation modifies the electronic properties of the graphite particles and, it is believed, affect the reaction of species on the surface of the catalyst particles. The mechanism of this is not yet understood, nor can it be said with any certainty what chemical and physical form the catalytic substance(s) on the surface of the catalyst takes. It is thought that groups or domains of the guest substance are ejected during de-intercalation and some studies indicate that fairly uniform particles, for example of up to about 175 Angstrom units diameter, are dispersed over the surface and in the surface micropores of the graphite particles. Published studies favour the explanation that the catalytic activity of intercalates depends upon geometrical considerations in the interstitial regions occupied by the guest substance, but the activity of the catalysts of the present invention in which 90% or more of the guest substance has been removed appears to contradict this theory and it is now thought that surface activity, with the electronic properties the disordered residually intercalated compound plays the major part in determining the catalytic properties.

It should be emphasised that the catalysts of the invention are distinct in form and activity from catalysts prepared by the conventional technique of impregnation of carbon particles, principally because of the residual disordered intercalation and the manner in which the expelled guest is modified. The important role that this residual intercalation plays may be demonstrated by heating the catalyst to a temperature of around 400°C, at which temperature the guest substance can escape entirely, or by complete de-intercalation, after which the particles, although apparently identical, are much less active and rather different in selectivity.

The de-intercalation process may be carried out in several ways; the method adopted depends upon whether the intercalate is a donor or acceptor type. Donor intercalates may be treated with a fluid at low temperature, and the fluid may be for example methanol, benzene, tetrahydrofuran, liquid ammonia or water. The preferred process for donor intercalates involves hydrolysis of the intercalate by the cooling of the particles to a temperature at which water vapour will deposit as ice on the surface thereof, followed by the gradual warming of the particles. Hydrogen gas evolves and the resulting catalyst may be recovered, for example by filtration, and dried, for example in air at about 100°C. The intercalate reacts explosively, however, with liquid water or with hydrogen at elevated temperature, and such treatments should be avoided. Straightforward heating of the donor intercalate in a vacuum can result in the complete loss of the intercalate metal, and is not recommended. The resulting catalyst is, contrary to the donor intercalate itself, extremely stable to exposure to air. In the case of a potassium intercalate processed according to the method of the invention, it is thought that one substance dispersed over the surface of the particles is potassium oxide. Exposure to air can cause reaction of the oxide with carbon dioxide, and a change in the surface morphology may be observed although there does not appear to be any change in the catalytic activity.

Acceptor intercalates generally require to be reduced to remove the metal ligands which interact with the graphite substrate. Reduction may be achieved by heating the intercalate in hydrogen at atmospheric pressure to a temperature of 300 to 500°C. The rate of heating is believed to be very important and to influence the size of the catalytic particles retained on the surface, and is suitably in the range 4 to 20°C/min. It is also important that the graphite be truly intercalated prior to this reduction steps. The intercalate may also be reduced by treatment with a liquid reducing agent including boranes and organic agents including especially alkaline naphthalenide. Preferably, the reducing agent is used in a very dilute solution in an organic solvent, to avoid the intercalation of the reducing agent itself, which could block further reaction by polymerisation. Reduction preferably carried out using an alkali metal, especially potassium, or alkaline earth metal naphthelenide in solution in an anhydrous solvent such as tetrahydrofuran. Such treatment also results in the incorporation of species derived from the alkali metal or alkaline earth metal in the catalyst, and this has been found to promote the catalyst and beneficially affect its selectivity.

Some acceptor intercalates, for example a ferric chloride intercalate, may be partially deintercalated according to the present invention by heating alone.

The catalysts of the invention are believed to represent a new class of catalysts. Some members of the class have been found in initial tests to give good activity, high selectivity and extended life, which could not have been predicted from the rather poor activity and life time of previously suggested graphite intercalate catalysts. A wide variety of catalysts may be prepared within the scope of the present invention, and while the activity shown will vary, from catalyst to catalyst, this can be easily determined by experiment. Initial tests indicate also that catalytic activity is shown at relatively low reaction temperatures, and that the formation of hydrocarbons, especially lower and unsaturated hydrocarbons from synthesis gas is catalysed by certain of the catalysts according to the invention. Accordingly, the invention further provides the use of the catalysts of the invention. The catalysts may be used alone or in combination with other catalysts or with diluents, in fixed bed, fluidised bed or dispersed phase systems. The stability of the catalysts of the invention to oxygen and water vapour is confirmed by unexpectedly long life in trials, indicating that an industrially practical catalyst system is possible. Preferably, however, the catalysts are not used at a temperature exceeding 250°C.

The invention will now be illustrated by way of example.

EXAMPLE

1mm flakes of natural graphite (Kropfmuhl grade S40) were intercalated by treatment at room temperature with a UV-irradiated saturated solution of ferric chloride in carbon tetrachloride for 24 hours, under a nitrogen atmosphere, to yield a first stage intercalate ($C_9$ $FeCl_3$). The intercalate was then reduced without delay, maintaining the nitrogen atmosphere, by treating with a 0.05M solution of potassium naphthelenide in anhydrous tetrahydrofuran. The product was separated, washed and dried, and analytical studies suggest that it consists of potassium promoted $\alpha-Fe_2$ $O_3$ supported on a disordered ferric chloride graphite residue intercalate. The ferric oxide particles appear, from scanning electron micrographs, to be on the surface of the particles and to be fairly uniform in size with a maximum diameter of about 175 Angstrom units. Analysis of the product gave a composition of 14.1% Fe, 19.6% Cl and 3.8% K, by weight, the balance being carbon. The product had a BET surface area of 3.9 $m^2/g$, indicating very little porosity.

The product catalyst was packed in a fixed bed in a tubular reactor and a mixture of hydrogen and carbon monoxide, 3:1 by volume, at one atmosphere total pressure, was passed over at a gaseous hourly space velocity of $ca$ $100h^{-1}$. The products were collected and analysed in conventional manner. Maintaining the reactor temperature at 100°C, approximately 20% carbon conversion was achieved, with approximately 95% selectivity to acetylene, the balance being methane and propane. At a reaction temperature of 200°C, selectivity to acetylene approached 100% but conversion dropped to below 10% after eight hours on stream and declined more slowly thereafter. Conversions at reaction temperatures of 300°C and 400°C were generally very low and a mixture of lower hydrocarbons with no acetylene was produced. The surprising selectivity to acetylene, and the percentage conversion at 100°C were maintained after one week on stream.

Instead of using potassium naphthelenide to reduce the ferric chloride intercalate, hydrogen gas was passed over the intercalate atmospheric pressure for 4 hours at temperatures up to 350°C, using heating rates varying from 4 to 40°C/min. Testing the resulting catalyst under the same condition as above yielded saturated hydrocarbons with no acetylene being detected. At a reactor temperature of 220°C, using a catalyst which had been reduced using a 4°C/min heating rate, greater than 90% selectivity to propane was demonstrated after 24 hours on stream, at a carbon conversion of about 50%.

0145478

PATENT CLAIMS:

1.   A polyphasic multicomponent heterogeneous catalyst characterised in_that is comprises particles of a disordered residue intercalate having on the outer surfaces of said particles a finely divided catalytic substance at least partially derived from the precursor guest substance.

2.   A catalyst according to claim 1, characterised in_that the disordered residue intercalate is a graphite intercalate.

3.   A catalyst according to claim 1 or 2, characterised in_that the guest substance is an alkali metal.

4.   A catalyst according to claim 3, characterised in_that the alkali metal is potassium.

5.   A catalyst according to claim 1 or 2, characterised in_that the guest substance is a compound of a metal in Group 4B, 5B, 6B, 7B and 8.

6.   A catalyst according to claim 5, characterised in that the guest substance is a compound of tin, antimony, nickel or iron.

7.   A catalyst according to claim 5 or 6, characterised in_that the compound is an oxide, sulfide or a halide.

8.   A method for the production of a catalyst according to claim 1, characterised in_that at least 90% by weight of the guest substance of an intercalate is deintercalated to yield a disordered residue intercalate carrying a finely divided catalytic substance at least partially derived from the guest substance, on the outer surface of said particles, and to retain from 1 to 10% of the guest substance in the intercalate matrix.

9.   A method according to claim 8, characterised in_that the starting material intercalate is a graphite intercalate.

10.   A method according to claim 9, characterised in that a donor intercalate is partially deintercalated using a fluid at low temperature.

11.    A method according to claim 10, characterised in that water vapour is deposited as ice on the intercalate particle surface, and the particles are gradually warmed.

12.    A method according to claim 9, characterised in that an acceptor intercalate is partially deintercalated by reduction using gaseous hydrogen, a borane or an alkaline naphthalenide.

13.    A method according to calim 9, characterised in that an acceptor intercalate is partially deintercalated by heating.

14.    The use of a catalyst according to any one of claims 1 to 7, in the conversion of systhesis gas to hydrocarbons.